# EUROPEAN PATENT SPECIFICATION

(11) **EP 2 196 158 B1**
(45) Date of publication and mention of the grant of the patent: **31.08.2011**
(21) Application number: 09178488.4
(22) Date of filing: 09.12.2009
(51) Int. Cl.: A61B 17/122, A61B 17/128

(54) **Multiple clip package**
Mehrfachklipppackung
Paquet à pinces multiples

(30) Priority: 10.12.2008 JP 2008314516
(43) Date of publication of application: 16.06.2010
(73) Proprietor: FUJIFILM Corporation, Minato-ku Tokyo (JP)
(72) Inventor: Matsuoka, Yoshiaki, Tokyo (JP); Oosako, Ryousuke, Osaka (JP); Ito, Koji, Tokyo (JP); Iida, Takayuki, Tokyo (JP)
(74) Representative: Klunker . Schmitt-Nilson . Hirsch

(56) References cited:
- US-A- 6 059 799
- US-A1- 2006 085 015
- US-A1- 2006 155 308
- US-B1- 6 306 149

## Description

### FIELD OF THE INVENTION

The present invention relates to a multiple clip package composed of a multiple clip unit to be loaded in a sequential clipping instrument and a clip case for encasing this multiple clip unit to provide hemostasis, wound closure and other treatments in succession.

### BACKGROUND OF THE INVENTION

A clip treatment is an endoscopic medical procedure. The clip treatment is performed with a clipping instrument that is inserted into a body of a patient through a forceps channel of an endoscope. A distal end of the clipping instrument is provided with a clip to be ejected from the endoscope for hemostasis and wound closure of an affected area, such as a bleeding site or a tumor removal site. A typical clipping instrument includes an operating wire for controlling the clip, and this operating wire is generally covered with a sheath. Japanese Patent Laid-open Publication No. 2007-222649 discloses a method of sheathing a clip unit having a coupling piece to engage with a rear end of the clip. This clip unit is enclosed in a clip case, and connected to the operating wire that extends through the sheath. The operating wire has a hook on its distal end to catch the clip unit.

The Publication No. 2007-222649 also discloses a push sleeve for closing the front end of the clip to mesh claws on the tip thereof. The push sleeve is put on the rear end of the clip, and loaded into the sheath. In the clip treatment, the push sleeve slides forward to close the front end of the clip. The push sleeve has skirts on the outer surface which have been closed in the sheath. Upon the forward slide and projection of the push sleeve out of the sheath, these skirts expand elastically in a radial direction to catch a distal end of the sheath, preventing the return of the clip into the sheath.

The Publication No. 2007-222649 further discloses a clip case for enclosing an unused clip together with the push sleeve. To maintain their elasticity, the skirts of the clip are kept opened in the clip case. Additionally, the clip case is provided with a sloping surface that leads to a clip outlet port and closes the skirts. The operating wire is inserted into the clip case through the clip outlet port, and the hook of the operating wire is attached to the clip. The operating wire is then pulled out of the clip case to load the clip with the push sleeve into the sheath. Along the way into the sheath, the skirts are forced to close by the sloping surface.

The above clipping instrument is configured to hold a single clip at a time. Accordingly, for every clip treatment, the clipping instrument has to be removed from the forceps channel of the endoscope, and load a next clip, and then be inserted again into the endoscope.

To avoid this complicated work, a clipping instrument of sequential type is introduced which can hold several clips at once and repeat the clip treatment in succession. For example, Japanese Patent Laid-open Publication No. 2006-87391 discloses a multiple clip unit composed of a train of clips. For every clip treatment, the foremost clip is pushed out of the clip unit and used.

The multiple clip unit includes a lot of elastic elements, such as a coupling piece for connecting the clips together. To maintain the elasticity of these elastic elements, the multiple clip unit is encased in a manner not to stress the elastic elements. In loading in the sheath, the multiple clip unit is once loaded, or transferred to a special loading jig, and then loaded into the sheath from the loading jig. This process allows for keeping each clip in good condition in the clip case until just before use.

Generally, the loading jig is attached to the multiple clip unit in the factory so as to ship them as a clip package. This clip package needs to ensure smooth insertion, or transfer, of the clip case into the loading jig, and secure fastening of the clip case to the loading jig during the transfer of the multiple clip unit in the loading jig. In view of this, the loading jig is provided with an insertion channel for receiving the clip case. This insertion channel has an open top to expose a part of the clip case. In transferring the multiple clip unit to the loading jig, the clip case is firmly attached to the loading jig by holding the exposed part of the clip case together with the loading jig.

However, inadequate holding may case the clip case to move up and down during the loading of the multiple clip unit, and the clip case becomes out of alignment with the loading jig. This condition makes it difficult to load the multiple clip unit in the loading jig. Also, in this condition, an external force may act on the clip unit in loading, and break or degrade the clips.

Document US-A-6059799 discloses a device which does not contain a loading jig as disclosed in the present invention.

The above Publication is silent about the loading jig, and accordingly, does not mention the above clip package problem and its countermeasure.

### SUMMARY OF THE INVENTION

In view of the foregoing, it is an object of the present invention to provide a multiple clip package for properly positioning a clip case containing a multiple clip unit with respect to a loading jig while preventing vertical movement of the clip case in loading of the clip unit into the loading jig.

In order to achieve the above and other objects, a multiple clip package according to the present invention includes a clip unit, a cylindrical case, a loading jig and a position regulating mechanism. The clip unit is made by serially coupling a train of clips and a coupling assembly, and the coupling assembly is connected to a distal end of an operating wire extending throughout a sheath of an instrument. The case encloses the clip unit in a removable manner. The loading jig has an insertion channel into which the case is inserted along its axial direction, and a passage for enclosing the clip unit after the removal from the case. The insertion channel has an opening on the top for exposing a part of the case. The passage extends concentrically with the case after the insertion in the insertion channel. The position regulating mechanism is composed of at least one rib formed in one of the case and the loading jig, and at least one engaging groove formed in the other one of the case and the loading jig to extend parallel to the insertion channel. This engaging groove is configured to engage with the rib to restrict vertical movement of the case within the insertion channel.

In a preferred embodiment of the present invention, the rib is formed to project laterally outward from a side of the case, and the engaging groove is hollowed out laterally from a side of the insertion channel.

Preferably, the rib is integrated with a front or rear end of the case.

The multiple clip package in the preferred embodiment also includes an upper case and a lower case. The upper case has a push plate integrated therewith for pushing the case against the loading jig. The ribs are formed on the lower case.

The multiple clip package further includes a key projecting from an underside of the case, and a key groove in the insertion channel. The key groove receives the key to prevent axial rotation of the case.

According to the present invention, the insertion channel positions the clip case with respect to the loading jig. The position regulating groove prevents the vertical movement of the clip case within the insertion channel. Accordingly, the clip unit is loaded, or transferred, properly into the loading jig from the clip case. Additionally, the clip unit is free from a bending force or other external forces, which may possibly damage and degrade the clip unit.

The ribs in the front and rear ends of the clip case prevent the clip case from lifting its front end even if the clip case is held on its rear end or if the clip case has warped. Furthermore, the engagement of the key and key groove restricts the axial rotation of the clip case. Thereby positioned in the axial rotation direction, the clip case can slide without difficulty, and is effectively protected from damage and degradation.

### BRIEF DESCRIPTION OF THE DRAWINGS

The above objects and advantages of the present invention will become more apparent from the following detailed description when read in connection with the accompanying drawings, in which:
FIG. 1 is a perspective view of a sequential-type clipping instrument according to the present invention;
FIG. 2A and FIG. 2B are cross-sectional views showing a front end of a sheath of the clipping instrument;
FIG. 3 is a perspective view of a clip and a coupling ring;
FIG. 4 is a cross-sectional view of the coupling ring;
FIG. 5 is a perspective view showing a rearmost hemostatic clip, a coupling clip assembly and an operating wire which are coupled to one another;
FIG. 6A to FIG. 6D are partial cross-sectional views showing the steps of a clip treatment using the clipping instrument;
FIG. 7 is a perspective view of a multiple clip package;
FIG. 8 is a cross-sectional view of the multiple clip package;
FIG. 9 is an exploded perspective view of the multiple clip package;
FIG. 10 is a perspective view showing a multiple clip unit, a clip case and a clip towing member;
FIG. 11 is an exploded perspective view of a loading jig;
FIG. 12 is a perspective view showing a case insertion channel and its surrounding in the loading jig;
FIG. 13A and FIG. 13B are cross-sectional views showing the procedure for loading the multiple clip unit in the loading jig from the clip case, and pulling out the clip case from the loading jig;
FIG. 14A and FIG. 14B are cross-sectional views showing the procedure for inserting the sheath into the loading jig, and coupling the operating wire to the multiple clip unit; and
FIG. 15A and FIG. 15B are cross-sectional views showing the procedure for loading the multiple clip unit from the loading jig into the sheath.

### DETAILED DESCRIPTION OF THE PREFERRED EMBODIMENTS

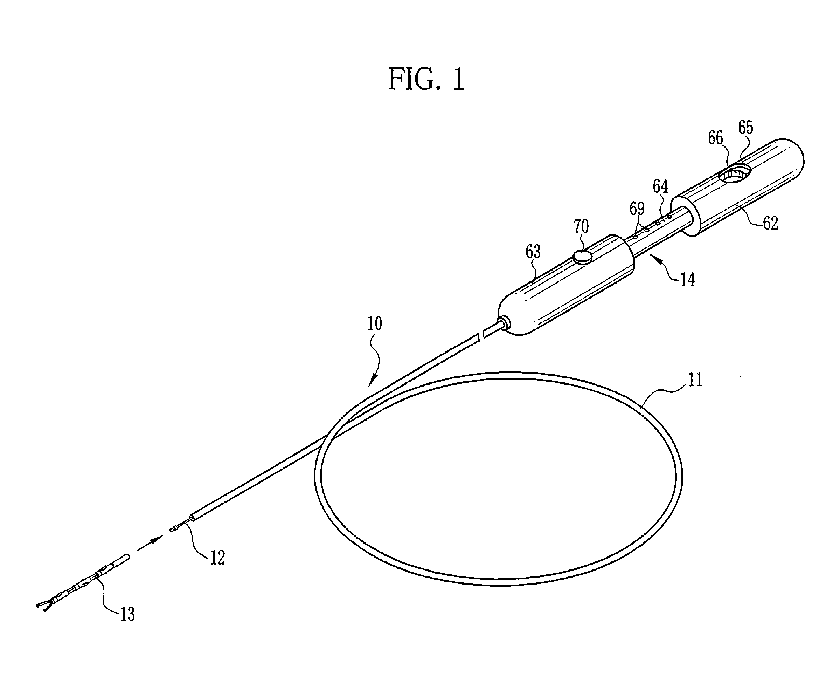

Referring to FIG. 1, a sequential-type clipping instrument, or clip applicator (hereinafter, clipping instrument) 10 includes a cylindrical sheath 11, an operating wire 12 extending through the sheath 11, a multiple clip unit (hereinafter, clip unit) 13 coupled to the operating wire 12 within the sheath 11, and an handling section 14 for pulling the sheath 11 and the operating wire 12. The sheath 11 is a, for example, flexible coil sheath of a tightly wound metal wire. The operating wire 12 is a wire of biocompatible metal.

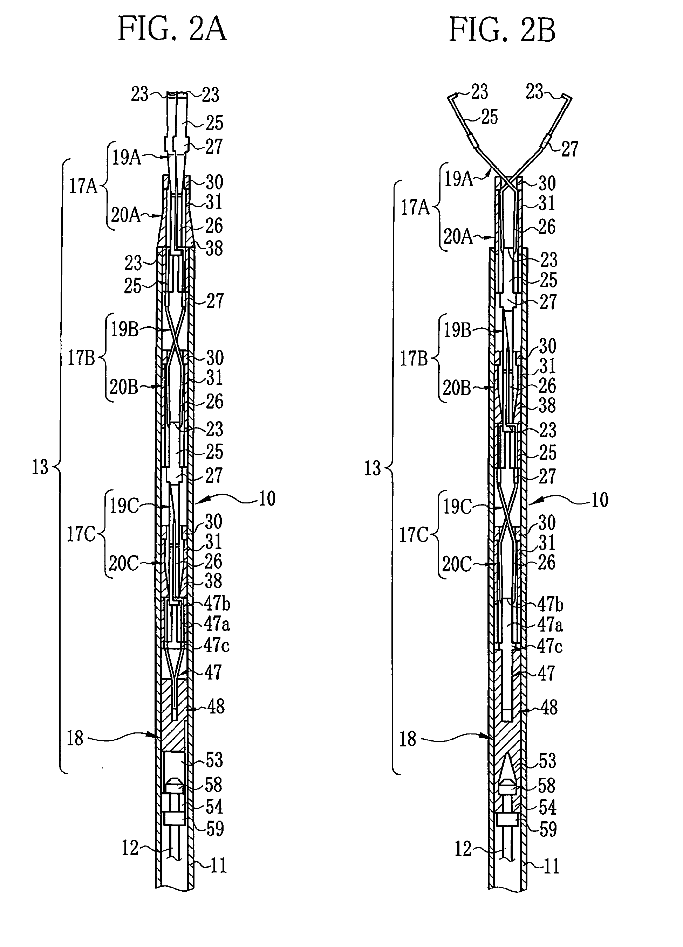

As shown in FIG. 2A and FIG. 2B, the clip unit 13 includes a train of hemostatic clip assemblies 17A-17C and a coupling clip assembly 18 connected to the rearmost hemostatic clip assembly 17C. The hemostatic clip assembly 17A has a clip 19A and a tubular coupling ring 20A for holding the rear end of the clip 19A. Similarly, the hemostatic clip assembly 17B has a clip 19B and a coupling ring 20B, and the hemostatic clip assembly 17C has a clip 19C and a coupling ring 20C. The clips 19A-19C share the same structure, and the coupling rings 20A-20C share the same structure.

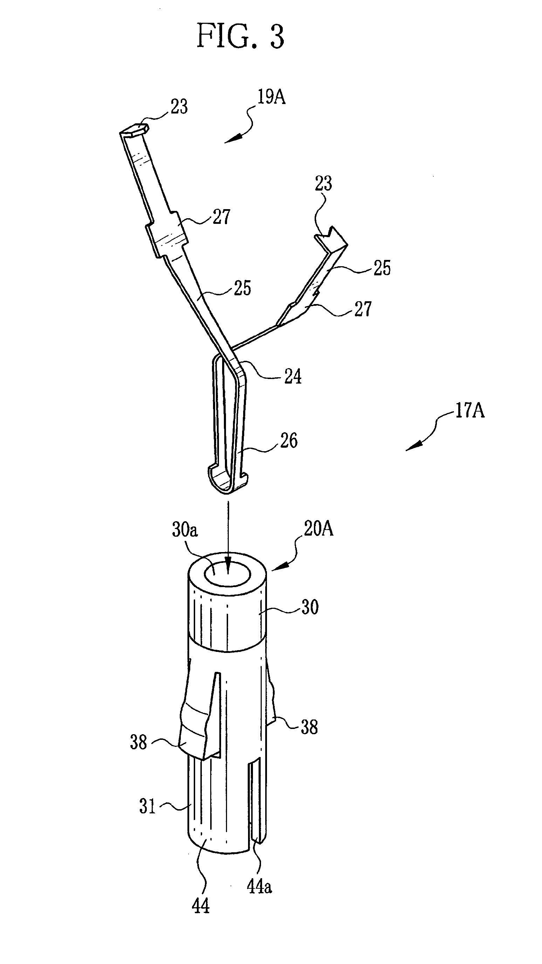

As shown in FIG. 3, the clip 19A is a Y-shaped element made by firstly bending a narrow plate into a U-shape to form a closed end, and then crossing both legs of this U-shaped plate. The ends of these legs are bent to form claws 23 that face to each other. Each leg of the clip 19A is divided at a crossover point 24 into two portions: an arm 25 on the open end side and a turn portion 26 on the closed end side. Each arm 25 has a wide portion 27 in the middle thereof.

Without an external force, the arms 25 remain in an open state to separate the claws 23. When an inward transverse force is applied thereto, the arms 25 elastically deform in a closed state to fasten the claws 23, allowing for holding an affected area in the clip treatment. To hold the affected area tightly, one of the claws 23 is made as a male claw that projects in a V-shape, and the other is a female claw that sinks in the V-shape. The clip 19A is preferably made of a biocompatible metal, and more preferably made of stainless steal SUS 631 that is a common material for springs.

The coupling ring 20A is a substantially cylindrical element that includes a metal-made fastening sleeve 30 in the front and a plastic-made retaining sleeve 31 in the rear. The fastening sleeve 30 has a fastening hole 30a passing therethrough. The clip 19A is inserted, from the side of the turn portion 26, into the fastening hole 30a, and the coupling ring 20A fits on the clip 19A to constitute the hemostatic clip assembly 17A. At this point, the fastening sleeve 30 rests in an initial position to cover the crossover point 24, and the clip 19A is in the open state.

When the clip 19A is pulled further into the coupling ring 20A, the arms 25 are closed by the fastening hole 30a. The arms 25 in the closed state provide the claws 23 with a certain meshing force. Since the diameter of the fastening hole 30a is smaller than the width of the wide portion 27, the front part of the clip 19A beyond the wide portion 27 does not enter the coupling ring 20.

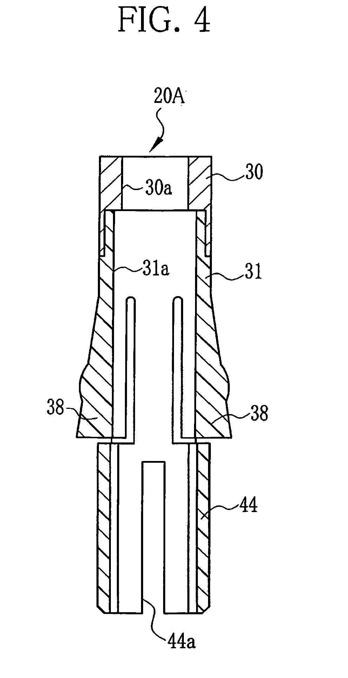

As shown in FIG. 4, the retaining sleeve 31 has a retaining hole 31a connected to the fastening hole 30a. The turn portion 26 passes through the fastening hole 30a, and enters the retaining hole 31a. The retaining sleeve 31 has two skirts 38 on the sides. Each of the skirts 38 tapers toward the front end of the retaining sleeve 31, and without an external force, opens to project outward from the retaining sleeve 31 because of its elasticity. When an inward transverse force is applied, the skirts 38 close to sink in the retaining sleeve 31.

The coupling ring 20A is attached to the clip 19A in such a manner that the arms 25 can open and close in the direction crosswise to the open/close direction of the skirts 38. Upon the insertion of the clip unit 13 into the sheath 11, the skirts 38 close to hold the turn portion 26 in the retaining hole 31a, so as to join the coupling ring 20A and the clip 19A.

In FIG. 2, the hemostatic clip assembly 17B engages its claws 23 with the turn portion 26 of the forward hemostatic clip assembly 17A. Similarly, the hemostatic clip assembly 17C engages its claws 23 with the turn portion 26 of the forward hemostatic clip assembly 17B. The coupling ring 20A ties the hemostatic clip assemblies 17A, 17B by covering the claws 23 of the rearward clip 19B. The wide portions 27 of the clip 19B, which are wider than the diameter of the retaining hole 31a, make contact with the rear end of the coupling ring 20A. Accordingly, the coupling ring 20A does not move rearward in the clip unit 13. The sheath 11 has an inner diameter so large as to release the engagement between the turn portion 26 of the clip 19A and the claws 23 of the clip 19B. In other words, the inner diameter of the sheath 11 is larger than the sum width of the two claws 23 and the turn portion 26.

As shown in FIG. 3, in a rear end 44 of the coupling ring 20A, or namely behind the skirts 38, there are two slits 44a displaced circumferentially by 90 degrees from the skirts 38. Extending in the axial direction of the coupling ring 20A, these slits 44a permit elastic deformation of the rear end 44, and improve the flexibility of the coupling ring 20A. Accordingly, the clip unit 13 has an ability to bend in accordance with the endoscope. The slits 44a also widen the inner diameter of the rear end 44, and facilitate covering the clip 19B in assembling the clip unit 13.

The retaining sleeve 31 is made of a biocompatible material that meets the elasticity and rigidity required to the skirts 38. An exemplary material for the retaining sleeve 31 is PPSU (polyphenylsulfone).

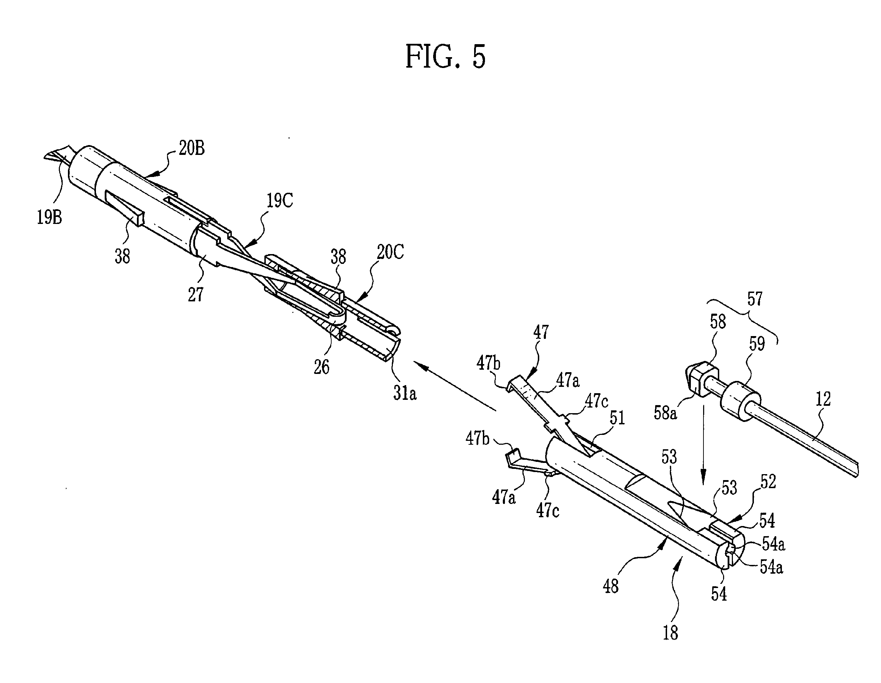

As shown in FIG. 5, the coupling clip assembly 18 includes a dummy clip 47 for holding the turn portion 26 of the rearmost clip 19C, and a coupling member 48 for supporting the dummy clip 47. The dummy clip 47 is made by bending a metal plate to form two openable arms 47a. Each of the arms 47a has a claw 47b for catching the turn portion 26, and a wide portion 47c to come into contact with the rear end of the coupling ring 20C. These arms 47a remain open because of their elasticity when no external force is applied, and are closed when pressed inwardly.

The coupling member 48 is a plastic-made columnar element having an outer diameter substantially equal to the coupling ring 20A. This coupling member 48 has a coupling part 52 to engage with the operating wire 12.

The operating wire 12 has a hook 57 on the distal end to engage with the coupling clip assembly 18. The hook 57 includes a front hook 58 on the tip of the operating wire 12 and a rear hook 59 on the downstream of the front hook 58.

The front hook 58 has a substantially quadrangular pyramid shape having a head to fit between elastic arms 53 of the coupling part 52 and a tail to make contact with a pair of nipping parts 54 of the coupling part 52. A part of the operating wire 12 between the front hook 58 and the rear hook 59 is nipped by the nipping parts 54, and thereby the slide and the rotational of the operating wire 12 are transmitted to the clip unit 13. This also supports the clip unit 13 from behind, and prevents it from moving rearward due to a frictional force of the sheath 11 in pulling.

Referring back to FIG. 1, the handling section 14 includes a wire handle 62 and a sheath handle 63 for pulling the operating wire 12 and the sheath 11 respectively. The sheath handle 63 is of cylindrical shape, and its front end is secured to the rear end of the sheath 11. The sheath handle 63 is slidably attached on a pipe 64 extending from the front end of the wire handle 62. Slide of the sheath handle 63 on the pipe 64 to the wire handle 62 leads to pull the sheath 11 rearward on the operating wire 12. A reference numeral 69 denotes notches that click to indicate the amount of slide of the sheath 11. These notches 69 are spaced apart by the distance to eject one clip from the sheath 11.

The wire handle 62 is of cylindrical shape, and has a through hole 65 for exposing a slidable operating lever 66. The operating wire 12 extends through the sheath handle 63 and the pipe 64, and engages with the operating lever 66 within the wire handle 62. The slide of the operating lever 66 leads to pull the operating wire 12 in the sheath 11 until the coupling ring 20A closes the clip 19A.

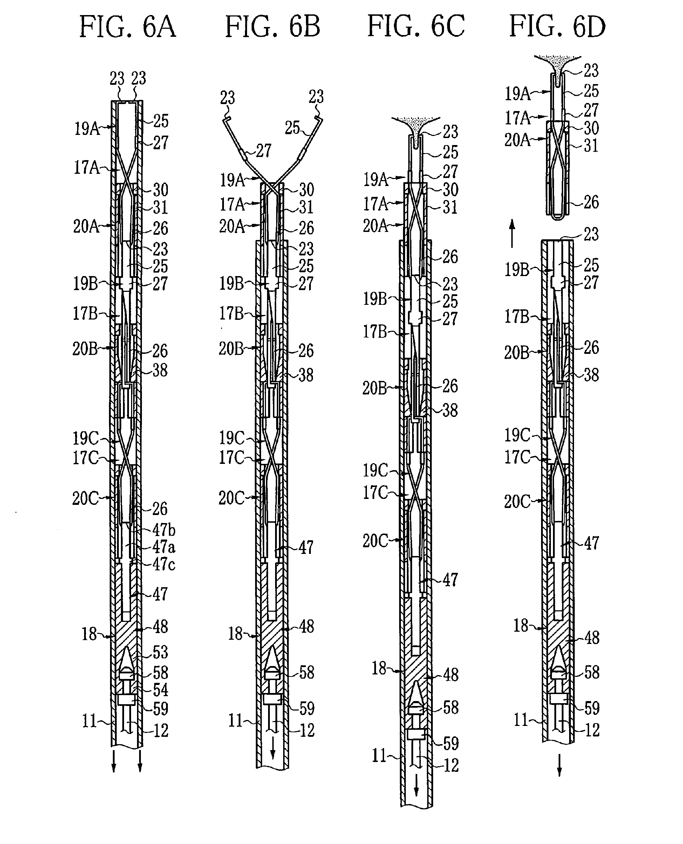

Next, the operation of the clipping instrument 10 is described. As shown in FIG. 6A, the clip unit 13 is loaded into the sheath 11 in a manner to align the front ends of the foremost clip 19A and the sheath 11. The sheath 11 is then inserted to the forceps channel of the endoscope that has been put in the body of a patient. The sheath 11 is advanced to project from the tip of the endoscope, and moved closer to an affected area.

The sheath handle 63 is pulled to move the sheath 11 rearward by a certain distance on the operating wire 12. Since the operating wire 12 is connected to the clip unit 13, as shown in FIG. 6B, the clip 19A and the coupling ring 20A come out from the front end of the sheath 11.

The rearward movement of the sheath 11 produces a frictional force to drag the coupling rings 20A-20C in contact with the inner wall of the sheath 11. The coupling rings 20A-20C are, however, secured to the clips 19A-19C by the skirts 38 in the closed state. In addition, the wide portions 27, 47c of the clips 19B, 19C and the dummy clip 47 restrict the rearward movement of the contents of the sheath 11. Accordingly, the coupling rings 20A-20C and the clips 19A-19C are hardly moved rearward.

The clip 19A out of the sheath 11 opens the arms 25 because of the elasticity (see, FIG. 2B) . Then, as shown in FIG. 2A, the skirts 38 of the coupling ring 20A expand beyond the inner diameter of the sheath 11, and prevents the coupling ring 20A from returning into the sheath 11.

The clipping instrument 10 is advanced to push the claws 23 of the already-opened clip 19A against the affected area. At this point, the operating lever 66 of the wire handle 62 is pulled to move the operating wire 12 rearward by a certain distance, and all the clips 19A-19C start moving rearward.

The operating wire 12 is kept pulled until the wide portions 27 of the clip 19A come into contact with the fastening sleeve 30. The clip 19A is closed by the coupling ring 20A, and the affected area is held by the claws 23.

At this point, or namely on completion of fastening the clip 19A, the engagement part of the clips 19A, 19B goes out of the coupling ring 20A. The arms 25 of the clip 19B open to touch the inner wall of the sheath 11 because of the elasticity, and the claws 23 are spaced apart wider than the width of the turn portion 26 of the clip 19A, whereby the clip 19A is disengaged from the clip 19B. Then, the sheath 11 is pulled away from the affected area to detach the hemostatic clip assembly 17A from the clipping instrument 10, and now, as shown in FIG. 6D, the hemostatic clip assembly 17A is left on the affected area.

The operating lever 66 is automatically returned to an initial position by a spring (not shown) as soon as it is released. Along with this, the operating wire 12 moves forward in the sheath 11 to push the coupling clip assembly 18 and the clips 19B, 19C until, as shown in FIG. 6D, the front end of the second clip 19B is aligned with the front end of the sheath 11. Thereafter, similar to the hemostatic clip assembly 17A, the handling section 14 is manipulated to repeat the clip treatments in succession using the hemostatic clip assemblies 17B, 17C.

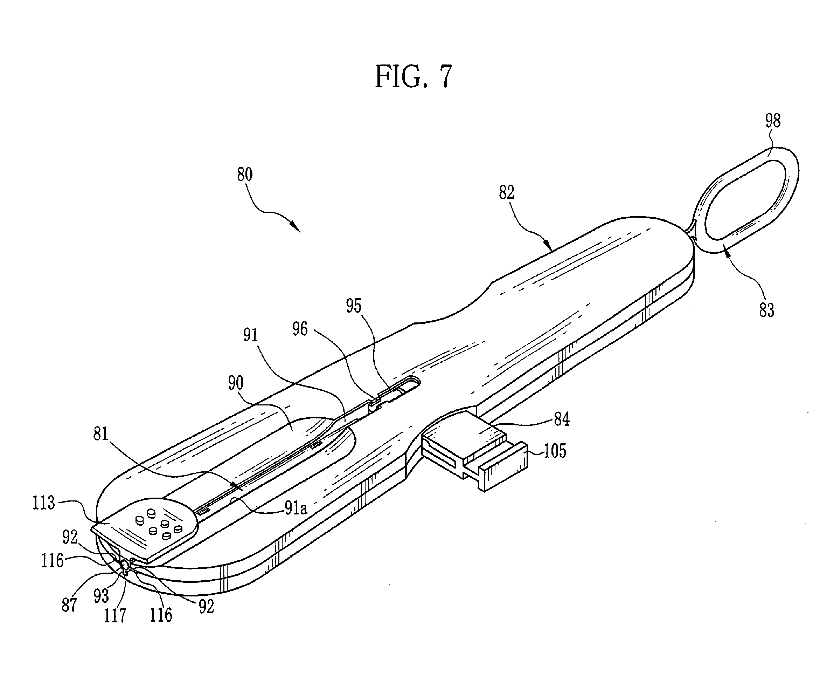

Next, a multiple clip package, or clip cartridge (hereinafter, clip package) 80 according to the present invention is described. As shown in FIG. 7 to FIG. 9, the clip package 80 includes a clip case 81 containing the clip unit 13, and a loading jig 82 for receiving the clip case 81. The loading jig 82 has a clip towing member 83 and a slider 84. The clip towing member 83 is used to pull out the clip unit 13 from the clip case 81 and load it into the loading jig 82. The slider 84 connects the operating wire 12 and the coupling clip assembly 18.

The clip case 81 has a cylindrical clip chamber 87. This clip chamber 87 extends longitudinally in the clip case 81. The clip case 81 has an outer diameter substantially equal to the outer diameter of the sheath 11, and the clip chamber 87 has an inner diameter substantially equal to the inner diameter of the sheath 11. The clip unit 13 is set in the clip chamber 87 in a manner to orient the foremost clip 19A to the front of the clip case 81 (or, namely, to the clip towing member 83).

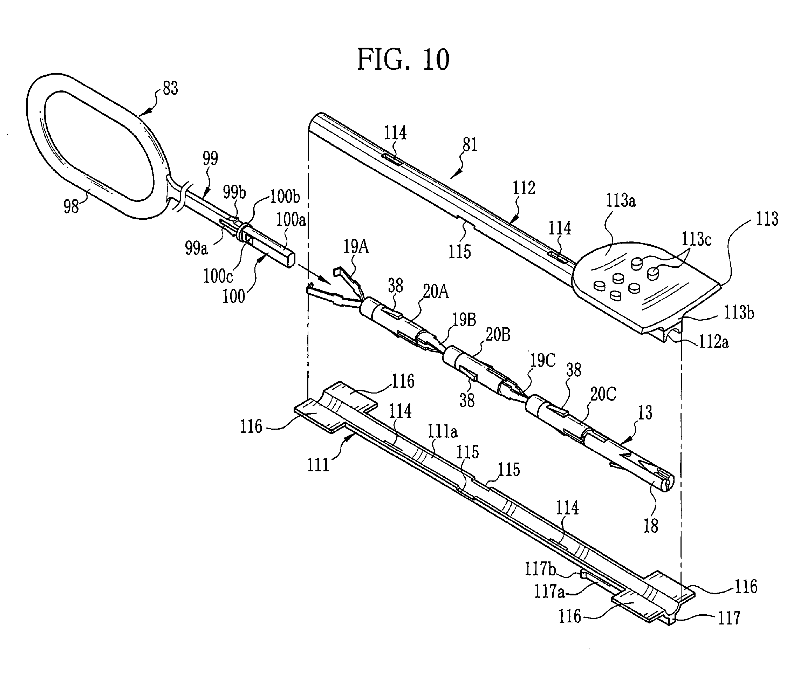

As shown in FIG. 10, the clip case 81 is composed of lower and upper cases 111, 112 (case components) of semicylindrical shapes. The lower and upper cases 111, 112 have recessed chambers 111a, 112a of semicircular cross-section. Upon the join of the lower and upper cases 111, 112, these recessed chambers 111a, 112a form the clip chamber 87. The clip chamber 87 has open ends.

The rear end of the upper case 112 is integrated with a push plate 113. The push plate 113 includes a push surface 113a that curves inwardly to follow the shape of a finger, and a leg 113b that extends in the axial direction of the upper case 112 to connect the push surface 113a to the upper case 112. The push surface 113a is provided with a plurality of anti-slip bumps 113c.

The clip case 81 is made of a transparent plastic material so that the clip unit 13 can be seen from the outside. The lower and upper cases 111, 112 are joined together by any conventional method, such as adhesive bonding, ultrasonic welding or use of engaging claws. Alternatively, the lower and upper cases 111, 112 may be combined by enclosing them with a transparent film.

The clip unit 13 is set in the clip chamber 87 to allow the arms 25 of the clip 19A to open horizontally. The clip 19A is pushed inwardly by the inner wall of the clip chamber 87, and stays in the closed state. As described, the hemostatic clip assemblies 17A-17C are coupled to one another in such a manner that the arms 25 of two successive clips open in the mutually perpendicular directions, and that the arms 25 and the skirts 38 open in the mutually perpendicular directions. In other words, the skirts 38 of the coupling rings 20A, 20C open vertically, and the skirts 38 of the coupling ring 20B open horizontally.

The lower case 111 and the upper case 112 have skirt open holes 114 for opening the skirts 38 of the coupling rings 20A, 20C. Along the joint line of the lower and upper cases 111, 112, there are provided cut-outs 115 that form horizontal skirt open holes. The skirt open holes permit the skirts 38 to open in the clip case 81, and thereby maintain the elasticity (the elastic restoring force in the opening direction) of the skirts 38. The skirt open holes may not necessarily be through holes but may be recesses.

The lower case 111 is integrated with four ribs 116 and a positioning key 117. The ribs 116 are rectangular plates projecting laterally outward from the front and rear ends of the lower case 111. Each of the ribs 116 is arranged in such a manner that its upper surface is flush with the joint line to the upper case 112. In other words, the ribs 116 are disposed below the axis of the clip case 81 after the joint of the lower and upper cases 111, 112.

The positioning key 117, which is attached to the underside of the rear end of the lower case 111, includes a leaf spring 117a extending from a rear end of the lower case 111. The leaf spring 117a has a fixed end on the rear side of the lower case 111 and a free end on the front side of the lower case 111, and is elastically swingable horizontally on the fixed end. The leaf spring 117a has a semicircular click-stop projection 117b on the free end.

The loading jig 82 is a device for temporarily receiving the clip unit 13 from the clip case 81, and then loading the clip unit 13 into the sheath 11. The loading jig 82 has a recess 90 of semicircular cross-section on the top surface. This recess 90 extends longitudinally from the tail end (the opposite end to the clip towing member 83) of the loading jig 82. In the middle of the recess 90, there is extended a case insertion channel 91 for receiving the clip case 81 and the sheath 11.

The case insertion channel 91 has a cross-section to fit onto the clip case 81. The case insertion channel 91 has an inner diameter slightly larger than the outer diameter of the clip case 81, and includes a channel slit 91a for exposing the upsides of the clip case 81 and the sheath 11. This channel slit 91a allows for holding the clip case 81 or the sheath 11 together with the loading jig 82.

In the case insertion channel 91, there are formed position regulating grooves (engaging grooves) 92 and a positioning key groove 93. The position regulating grooves 92 extend along the side edges of the case insertion channel 91. The positioning key groove 93 is disposed on the bottom of the case insertion channel 91, and elongated from the tail end of the loading jig 82. Upon the insertion of the clip case 81 into the case insertion channel 91, the position regulating grooves 92 receive the ribs 116 of the clip case 81, and the positioning key groove 93 fits onto the positioning key 117. After the transfer of the clip unit 13 into the loading jig 82, the clip case 81 is removed from the case insertion channel 91, and the sheath 11 is inserted.

The loading jig 82 also has a coupling hole 95 and a wire slit 96 behind the recess 90. The coupling hole 95 is larger than the dimension of the hook 57 of the operating wire 12. The wire slit 96 connects the coupling hole 95 and the case insertion channel 91, and has a width to tightly hold the operating wire 12. To insert the sheath 11 in the case insertion channel 91, the operating wire 12 is inserted to the wire slit 96. Then, the hook 57 is pushed into the coupling hole 95 until it engages with the coupling member 48 of the coupling clip assembly 18 in the loading jig 82 (see, FIG. 5).

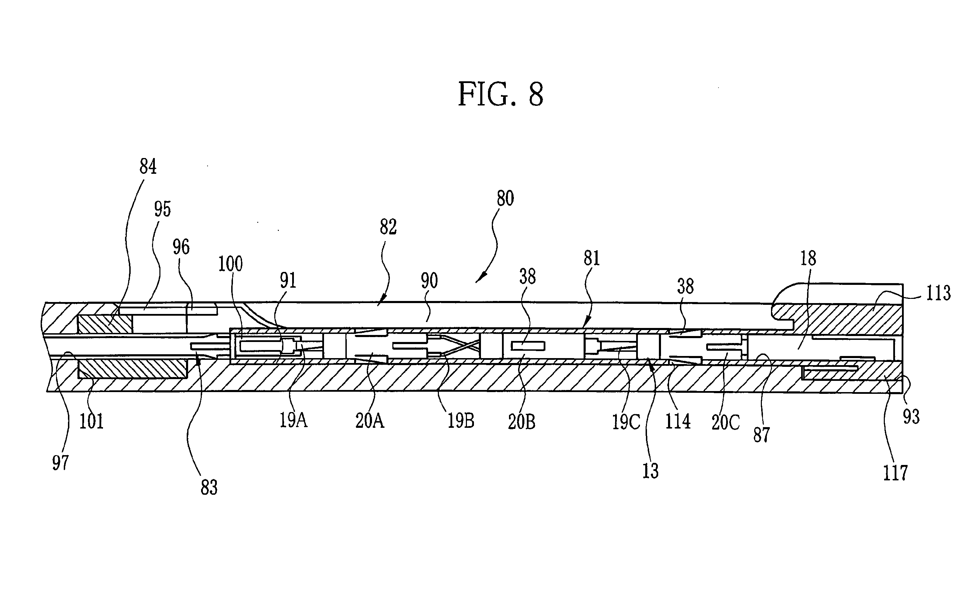

As shown in FIG. 8, the case insertion channel 91 leads to a cylindrical skirt closing passage 97. The skirt closing passage 97 has an inner diameter substantially equal to the inner diameters of the sheath 11 and the clip chamber 87. Upon the insertion of the clip case 81 into the case insertion channel 91, the skirt closing passage 97 comes into line with the clip chamber 87, forming a continuous inner wall with the clip chamber 87. The skirt closing passage 97 closes the skirts 38 of the clip unit 13 passing therethrough from the clip chamber 87.

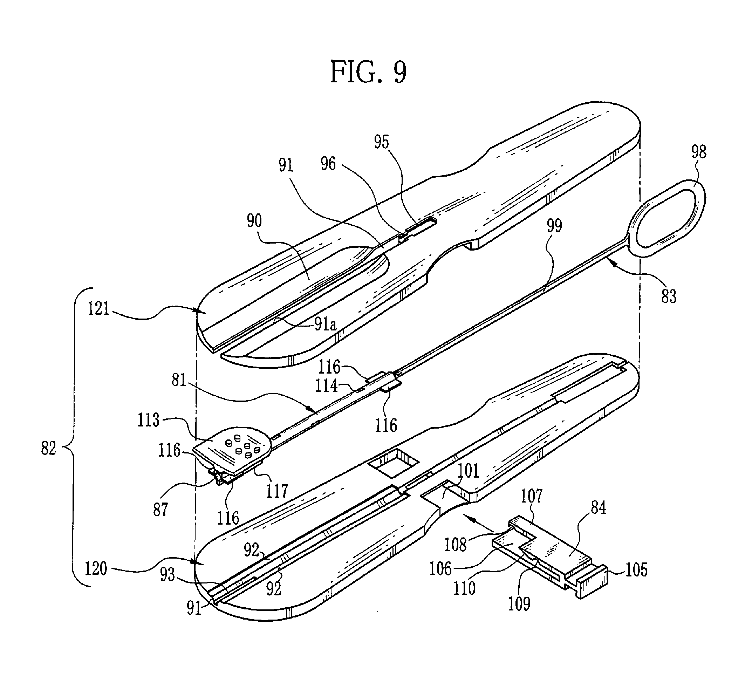

As shown in FIG. 9 and FIG. 10, the clip towing member 83 includes a substantially elliptical tow ring 98 that projects longitudinally from the front end of the loading jig 82, a penetrating portion 99 coupled to the tow ring 98, and an engaging portion 100 connected to a distal end of the penetrating portion 99. These tow ring 98, penetrating portion 99 and engaging portion 100 are formed integrally of elastic plastic material or the like. Alternatively, the penetrating portion 99 and the engaging portion 100 may be formed separately and then joined together.

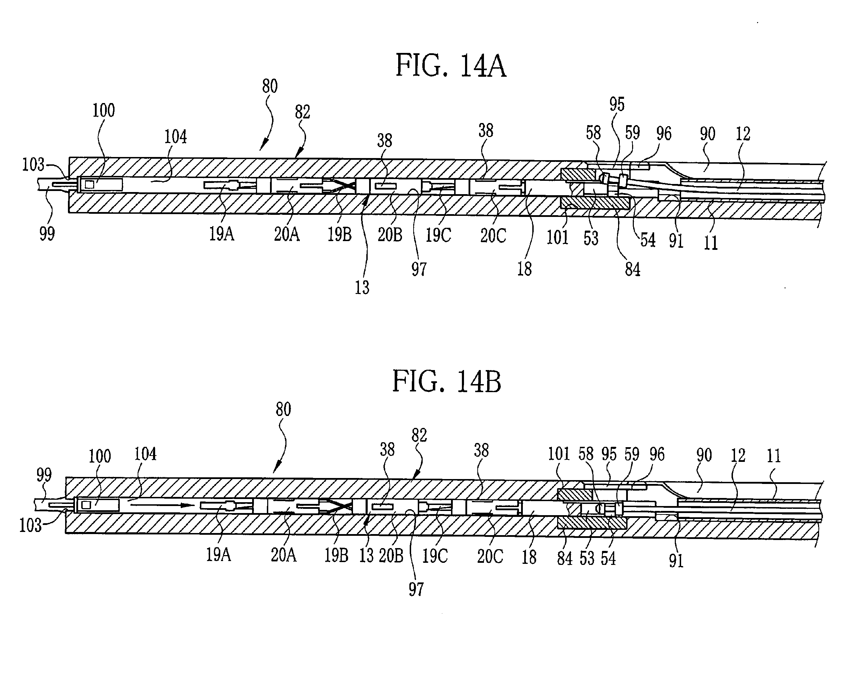

The penetrating portion 99 is a rod-like element longer than the skirt closing passage 97, and is inserted in the skirt closing passage 97 from a tow opening 103 (see, FIG. 14 to FIG. 16). A slit 99a is formed in the penetrating portion 99. Above and below the slit 99a, there are provided non-return flaps 99b that are tapered from the engaging portion 100. Because of the slit 99a, the penetrating portion 99 can elastically deform at the one end. Upon the pull of the tow ring 98 to remove the penetrating portion 99 from the skirt closing passage 97, the non-return flaps 99b expand beyond the tow opening 103, and prevent the penetrating portion 99 from returning into the skirt closing passage 97.

The engaging portion 100 is inserted in the clip chamber 87, and held by the clip 19A. The engaging portion 100 includes an engagement piece 100a of substantially rectangular column, and a retaining flange 100b at the boundary to the penetrating portion 99. The engagement piece 100a has an engagement hole 100c for engaging with the claws 23. The retaining flange 100b has an outer diameter substantially equal to the inner diameter of the clip chamber 87. Configured to close the one end of the clip chamber 87, the retaining flange 100b prevents the clip 19A from falling out of the clip chamber 87.

The clip 19A remains in the closed state within the clip chamber 87, and its claws 23 hold the engagement hole 100c. Upon the pull of the tow ring 98, the penetrating portion 99 slides in the skirt closing passage 97 to move the engaging portion 100 from the clip chamber 87 to the skirt closing passage 97.

Along with the movement of the engaging portion 100, the clip unit 13 gradually enters the skirt closing passage 97, which is connected to a horizontally-wide releasing groove 104 (see, FIG. 14 to FIG. 16) . The releasing groove 104 has a width (or depth in the horizontal direction) to allow the arms 25 of the clip 19A to open and release the engagement portion 100. The clip 19A enters into the releasing groove 104 as the coupling part 52 of the coupling clip assembly 18 faces the coupling hole 95.

When the clip 19A reaches the releasing groove 104, the arms 25 open to release the engagement portion 100. The clip unit 13 stops in the skirt closing passage 97, and the coupling part 52 of the coupling clip assembly 18 enters a slider channel 101.

Since the hemostatic clip assemblies 17A-17C are arranged to open/close the arms 25 in the direction perpendicular to the open/close direction of the skirts 38, the skirts 38 of the coupling ring 20A remain closed even when the clip 19A opens.

The slider channel 101 extends in a transversal direction (perpendicular to the case insertion channel 91) of the loading jig 82, and receives the slider 84. The slider 84 is used for engaging the coupling clip assembly 18 with the hook 57 inserted in the coupling hole 95.

The slider 84 is composed of a base block 105, a bottom piece 106 and a top piece 107 which are formed integrally of plastic material. The base block 105 is seized or pushed in inserting the slider 84 into the slider channel 101. The bottom and top pieces 106, 107 extend horizontally parallel to each other from the base block 105, and hold the sheath 11 or the clip case 81 in the case insertion channel 91 from above and below.

An underside of the top piece 107 is provided with a first recess 108 and a second recess 109 that fit onto the sheath 11 and the clip case 81 respectively. The slider 84 is slidable between an initial position to place the first recess 108 in the skirt closing passage 97 and a coupling position to place the second recess 109 in the skirt closing passage 97.

The underside of the top piece 107 also has a guide slope 110 for coupling assist. Upon the slide of the slider 84 from the initial position to the coupling position, the guide slope 110 pushes the hook 57 downward to engage the hook 57 with the coupling member 48 of the coupling clip assembly 18.

When the hook 57 is inserted into the coupling hole 95, a prismatic end 58a of the front hook 58 overlaps the elastic arms 53 (see, FIG. 5) ofthecouplingmember48. Then, upon the insertion of the slider 84 in the slider channel 101, the guide slope 110 comes into contact with the front and rear hooks 58, 59. Pushed downward by the slider 84, the front hook 58 rotates on the guide slope 110 until it enters between the elastic arms 53. Along with this, the operating wire 12 enters between the nipping parts 54, and the rear hook 59 comes into contact with a tail of the nipping parts 54.

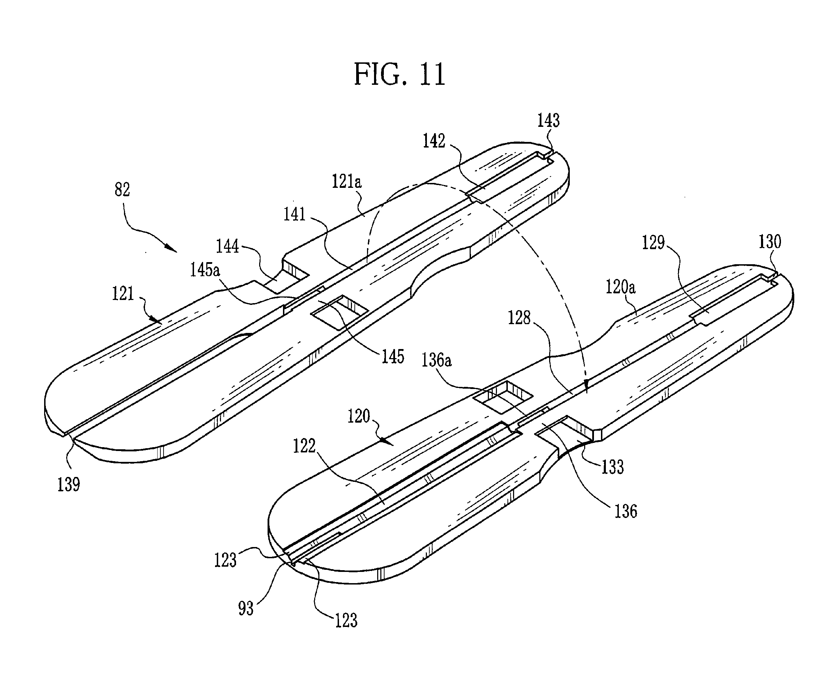

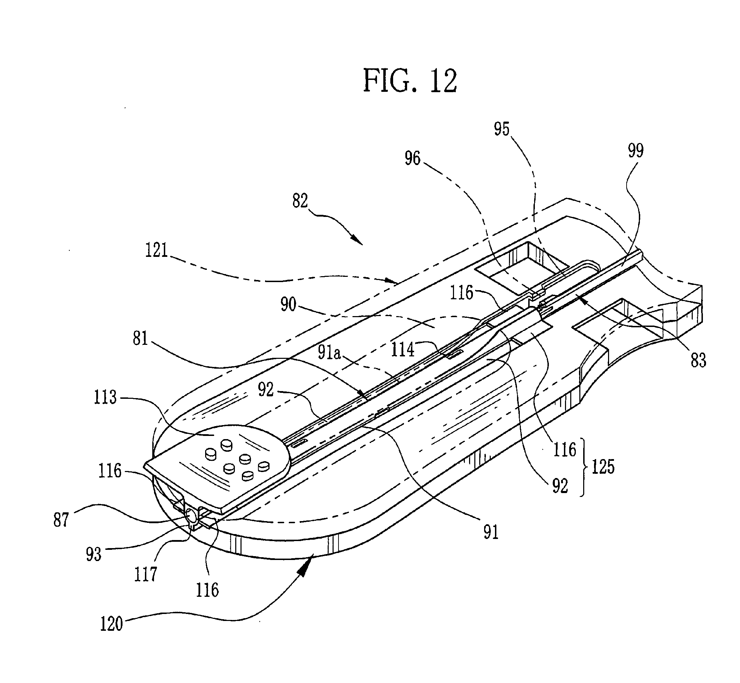

As shown in FIG. 11 and FIG. 12, the loading jig 82 is composed of plate-like lower and upper jigs 120, 121. The lower and upper jigs 120, 121 have front and rear ends of semicircular shape. A top surface 120a of the lower jig 120 is formed with a case groove 122 of arcuate cross-section that constitutes the case insertion channel 91 upon the joint of the lower and upper jigs 120, 121.

Along each side of the case groove 122, there is extended a position regulating recess 123 that sinks from the top surface 120a. Upon the joint of the lower jig 120 and the upper jig 121, the position regulating recess 123 is covered with a bottom surface 121a of the upper jig 121, and constitutes the position regulating groove 92. Namely, the ribs 116 and the position regulating grooves 92 constitute a position regulating mechanism 125 that prevents the clip case 81 from moving toward the channel slit 91a in the case insertion channel 91.

The positioning key groove 93, which extends on the bottom of the case groove 122 as described, holds the positioning key 117 to prevent the axial rotation of the clip case 81 when the clip case is inserted in the case insertion channel 91.

The positioning key groove 93 includes a click hole (not shown) of rectangular cross-section, which fits onto the click-stop projection 117b of the positioning key 117. The click-stop projection 117b enters the click hole as the positioning key 117 reaches a predetermined position in the case insertion channel 91. The click hole makes click-engagement with the click-stop projection 117b, and thereby fixes the axial position of the clip case, and prevents the withdrawal of the clip case 81 from the loading jig 82.

The case groove 122 leads to a skirt closing groove 128. The skirt closing groove 128 has an arcuate cross-section, and forms the skirt closing passage 97 upon the joint of the lower and upper jigs 120, 121. The skirt closing groove 128 is connected to a rectangular releasing recess 129, which is then connected to a rectangular cut-out 130 for receiving the penetrating portion 99 of the clip towing member 83.

A slider recess 133 is formed behind the case groove 122. The slider recess 133 extends perpendicular to the case groove 122. Upon the joint of the lower jig 120 and the upper jig 121, the slider recess 133 forms the slider channel 101 for guiding the slider 84. The slider recess 133 is of rectangular shape having a width substantially equal to the width of the slider 84, and elongated across the transverse direction of the lower jig 120.

The middle portion of the slider recess 133 is bridged by a plate-like connecting piece 136. This connecting piece 136 enhances the strength of the loading jig 82 which has been lowered by forming the slider recess 133, and also acts as a stopper for the slider 84 in the slider channel 101. An upside of the connecting piece 136 constitutes a part of the case groove 122, which has a coupling assist opening 136a passing vertically to the slider recess 133.

Similar to the lower jig 120, the bottom surface 121a of the upper jig 121 has a case cut-out 139 that constitutes the case insertion channel 91. The bottom surface 121a is also provided with a skirt closing groove 141 that constitutes the skirt closing passage 97, a releasing recess 142, and a cut-out 143 of the same shape as the cut-out 130. Additionally, disposed on the upstream side of the case cut-out 139 are a slider recess 144 that constitutes the slider channel 101, a connecting piece 145, and a coupling assist opening 145a. The coupling assist opening 145a faces the coupling hole 95 (see, FIG. 7).

The releasing recesses 129, 142 are combined, upon the joint of the lower jig 120 and the upper jig 121, to form the releasing groove 104 that expands laterally from the skirt closing passage 97. The releasing groove 104 has a width enough to allow the arms 25 of the front clip 19A to open and release the engaging portion 100 of the clip towing member 83. The cut-outs 130, 143 are combined to form the tow opening 103.

The loading jig 82 is made of transparent plastic material or the like so that the clip unit 13, the clip case 81, the clip towing member 83 and the slider 84 can be seen from the outside.

In assembling the clip package 80, the clip unit 13 is firstly set up by coupling the clips 19A-19C, the dummy clip 47 and the coupling member 48 on the lower case 111.

Then, the clip unit 13 on the lower case 111 is oriented to set the skirts 38 in the skirt open holes 114 and the cut-outs 115 (as better shown in FIG. 10) . The engaging portion 100 of the clip towing member 83 is put in the lower case 111, and the arms 25 of the front clip 19A are closed to engage the claws 23 with the engagement hole 100c. The lower case 111 is then covered with the upper case 112 to constitute the clip case 81.

The clip case 81 containing the clip unit 13 is disposed, together with the clip towing member 83, on the lower jig 120. Specifically, the clip case 81 is set in the case groove 122, and the clip towing member 83 is set in the skirt closing groove 128. At this point, the ribs 116 are in the position regulating recess 123. Thereafter, the lower jig 120 is covered with the upper jig 121 to complete the clip package 80.

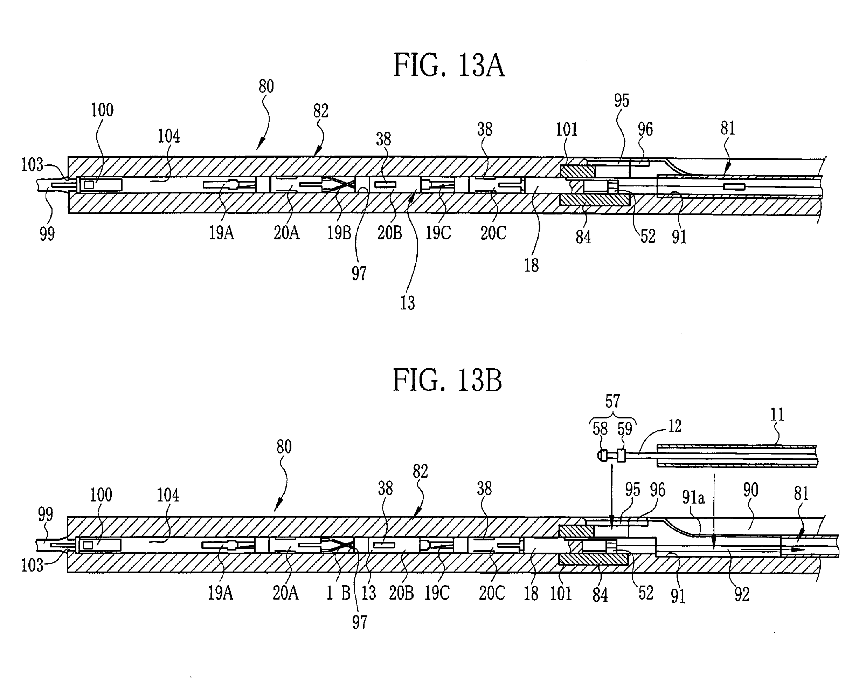

Next, with reference to FIG. 13 to FIG. 15, the process for loading the clip unit 13 into the sheath 11. Before the use of the clip package 80, as shown in FIG. 12, the ribs 116 are in contact with an end wall of the position regulating grooves 92. Additionally, the positioning key 117 fits in the positioning key groove 93, and the click-stop projection 117b engages with the click hole.

The clip case 81 can be secured to the loading jig 82 by placing a finger placed in the recess 90, and in this condition, the tow ring 98 is pulled away from the loading jig 82. It is preferred, at this point, to press down the push plate 113 with the same finger so as to secure the clip case 81 to the loading jig 82. The penetrating portion 99 is pulled out of the loading jig 82, and whereby the engaging portion 100 is moved to the skirt closing passage 97. Pulled by the engaging portion 100, the clip unit 13 enters from the clip chamber 87 into the skirt closing passage 97. The engagement of the ribs 116 and the regulating grooves 92 prevents the upward movement of the clip case 81, or namely, the movement toward the channel slit 91a, and aligns the clip case 81 with the skirt closing passage 97 in the loading jig 82. Accordingly, the clip unit 13 in towing is free from a strong force in the vertical direction, and thus no damage or deterioration is caused in the clip unit 13 before use.

As shown in FIG. 13A, the clip unit 13 is pulled into the skirt closing passage 97, and each of the skirts 38 of the coupling rings 20A-20C is closed by the inner wall of the skirt closing passage 97. When the front clip 19A reaches the releasing groove 104, the arms 25 of the clip 19A open to release the engagement of the claws 23 and the engaging portion 100. The clip unit 13 rests on a predetermined position in the skirt closing passage 97. In this resting position, the coupling member 48 of the coupling clip assembly 18 resides immediately below the coupling hole 95. The clip towing member 83 is pulled until the non-return flaps 99b go out of the loading jig 82.

Thereafter, the push plate 113 is pulled in the axial direction, and as shown in FIG. 13B, the clip case 81 is removed from the case insertion channel 91. Without the clip case 81, the coupling hole 95 and the wire slit 96 reveal a part of the coupling member 48 of the coupling clip assembly 18. The coupling part 52 of the coupling member 48 faces the coupling hole 95 as it enters the slider channel 101.

The sheath handle 63 is pulled to project the operating wire 12 and the hook 57 from the distal end of the sheath 11. The sheath 11 is then put into the case insertion channel 91 from above the loading jig 82. As shown in FIG. 14A, the operating wire 12 and the hook 57 are inserted in the coupling hole 95 and the wire slit 96 respectively. In this state, the sheath 11 can be firmly attached to the loading jig 82 by holding the recess 90 with a finger.

The slider 84 is inserted in the slider channel 101, and the guide slope 110 pushes the front hook 58 and the rear hook 59 downward. Held down by the slider 84, as shown in FIG. 14B, the front hook 58 is inserted into the elastic arms 53. At the same time, the operating wire 12 is pushed between the nipping parts 54. The rear ends of the nipping parts 54 come into contact with the rear hook 59.

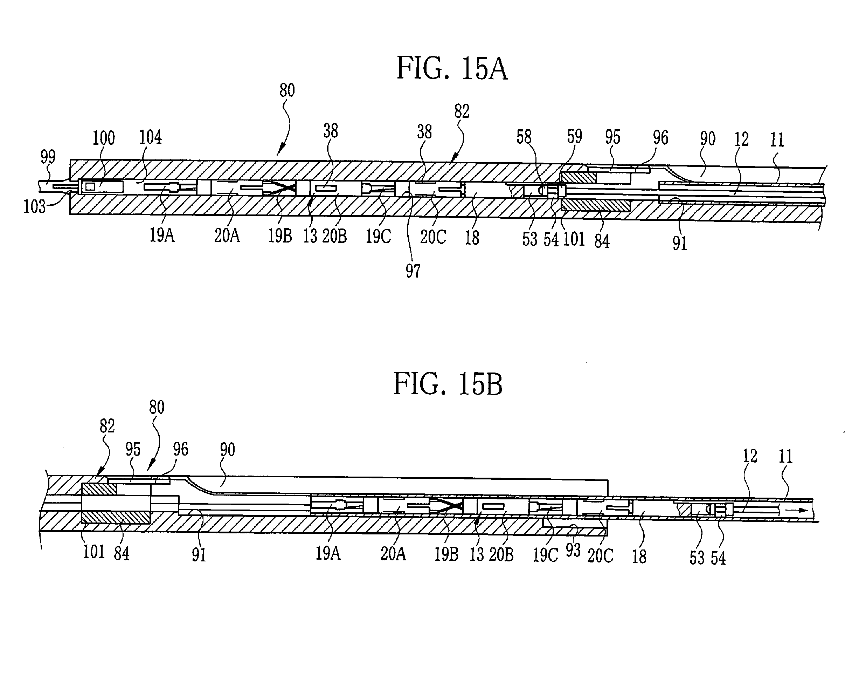

The clipping instrument 10 is now pushed forward to the loading jig 82, and whereby, as shown in FIG. 15A, the sheath 11 is inserted all the way into the case insertion channel 91, and the sheath 11 and the skirt closing passage 97 form the continuous inner wall. In this period, the clip unit 13 is pushed by the rear hook 59 to advance in the skirt closing passage 97.

Then, the wire handle 62 is pulled away from the sheath handle 63 to move the operating wire 12 rearward in the sheath 11.

As shown in FIG. 15B, this rearward movement of the operating wire 12 sends the clip unit 13 into the sheath 11. Since the inner surface of the sheath 11 continues from skirt closing passage 97, the skirts 38 are kept closed in loading the clip unit 13 into the sheath 11, resulting in lowering a pulling resistance. As a result, the relative positions of the clips 19A-19C and the coupling rings 20A-20C are maintained properly after the loading of the clip unit 13 into the sheath 11.

The sheath handle 63 is then pulled to the wire handle 62 until it engages with a first notch 69, and the clip unit 13 is completely loaded in the sheath 11. The loading jig 82 is now detached from the sheath 11, and the clipping instrument 10 is ready for the clip treatment.

The clip package 80 is applicable to not only the flexible endoscopes, but also rigid endoscopes.

Although the present invention has been fully described by the way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. A multiple clip package (80) for loading a train of clips (19A, 19B, 19C) into a sheath (11) of an instrument (10) comprising:
a clip unit (13) composed of said train of clips (19A, 19B, 19C) and a coupling assembly (18) attached to a rearmost said clip (19C), said coupling assembly (18) being connected to a distal end of an operating wire (12) extending throughout said sheath (11);
a cylindrical case (81) for removably enclosing said clip unit (13);
a loading jig (82) having an insertion channel (91) which receives axial insertion of said case (81) and has an opening (91a) on the top for exposing a part of said case (81), and a passage (97) which extends concentrically with said case in said insertion channel (91) and receives said clip unit (13) having been removed from said case (81); and
a position regulating mechanism (125) composed of at least one rib (116) being formed in one of said case (81) and said loading jig (82), and at least one engaging groove (92) being formed in the other one and extending parallel to said insertion channel (91), said engaging groove (92) configured to engage with said rib (116) to restrict vertical movement of said case (81) within said insertion channel (91).

2. The multiple clip package (80) of claim 1, wherein said rib (116) projects laterally outward from a side of said case (81), and said engaging groove (92) is hollowed out laterally from a side of said insertion channel (91).

3. The multiple clip package (80) of claim 2, wherein said rib (116) is integrated with a front or rear end of said case (81).

4. The multiple clip package (80) of any of claims 1 to 3, wherein said case (81) includes an upper case (112) and a lower case (111), and wherein said upper case (112) has a push plate (113) integrated therewith for pushing said case (81) against said loading jig (82), and said lower case (111) has said ribs (116).

5. The multiple clip package (80) of any of claims 1 to 4, further comprising:
a key (117) projecting from an underside of said case (81); and
a key groove (93) in said insertion channel (91), said key groove (93) receiving said key (117) to prevent axial rotation of said case (81).

## Patentansprüche

1. Mehrfachklipppackung (80) zum Laden einer Kette von Klipps (19A, 19B, 19C) in eine Hülle (11) eines Instruments (10) umfassend:
eine Klippeinheit (13), zusammengesetzt aus der Kette von Klipps (19A, 19B, 19C) und einer an einem hintersten Klipp (19C) befestigten Kopplungsanordnung (18), die mit einem distalen Ende eines Betätigungsdrahts (12) verbunden ist, der sich durch die Hülle (11) erstreckt;
ein zylindrisches Gehäuse (81) zum lösbaren Umschließen der Klippeinheit (13);
eine Ladelehre (82), die einen Einführkanal (91), der ein axiales Einführen des Gehäuses (81) aufnimmt und oben eine Öffnung (91 a) zum Freilegen eines Teils des Gehäuses (81) besitzt, und einen Kanal (97) aufweist, der sich konzentrisch zu dem Gehäuse in dem Einführkanal (91) erstreckt und die aus dem Gehäuse (81) entfernte Klippeinheit (13) aufnimmt; und
einen Positionsreguliermechanismus (125), zusammengesetzt aus mindestens einer Rippe (116), die in entweder dem Gehäuse (81) oder der Ladelehre (82) ausgebildet ist, und mindestens einer Eingriffsnut (92), die in der Ladelehre (82) bzw. dem Gehäuse (81) ausgebildet ist und sich parallel zu dem Einführkanal (91) erstreckt, wobei die Eingriffsnut (92) konfiguriert ist zum Zusammenwirken mit der Rippe (116), um eine Vertikalbewegung des Gehäuses (81) innerhalb des Einführkanals (91) zu beschränken.

2. Mehrfachklipppackung (80) nach Anspruch 1, bei der die Rippe (116) seitlich von einer Seite des Gehäuses (81) absteht, und die Eingriffsnut (92) seitlich von einer Seite des Einführkanals (91) ausgehöhlt ist.

3. Mehrfachklipppackung (80) nach Anspruch 2, bei der die Rippe (116) mit einem vorderen oder hinteren Ende des Gehäuses (81) integriert ist.

4. Mehrfachklipppackung (80) nach einem der Ansprüche 1 bis 3, bei der das Gehäuse (81) ein Obergehäuse (912) und ein Untergehäuse (111) enthält, von denen das Obergehäuse (112) eine mit ihm integriert ausgebildete Stoßplatte (113) zum Stoßen des Gehäuses (81) gegen die Ladelehre (81) aufweist und das Untergehäuse die Rippen (116) enthält.

5. Mehrfachklipppackung (80) nach einem der Ansprüche 1 bis 4, weiterhin umfassend:
eine von einer Unterseite des Gehäuses (81) abstehende Feder (117); und
eine Federnut (93) in dem Einführkanal, wobei die Federnut (93) die Feder (117) aufnimmt, um eine axiale Drehung des Gehäuses (81) zu verhindern.

## Revendications

1. Paquet à pinces multiples (80) pour charger un train de pinces (19A, 19B, 19C) dans une gaine (11) d'un instrument (10), comprenant :
une unité de pinces (13) composée dudit train de pinces (19A, 19B,
19C) et un ensemble de couplage (18) attaché à une pince (19C) tout à fait à l'arrière, ledit ensemble de couplage (18) étant connecté à une extrémité distale d'un fil d'actionnement (12) s'étendant à travers toute ladite gaine (11):
un casier cylindrique (81) pour enfermer de façon amovible ladite unité de pinces (13) ;
une monture de chargement (82) ayant un canal d'insertion (91) qui reçoit ledit casier (81) en insertion axiale et possède une ouverture (91a) au sommet pour exposer une partie dudit casier (81), et un passage (97) qui s'étend concentriquement avec ledit casier dans ledit canal d'insertion (91) et reçoit ladite unité de pinces (13) qui a été enlevée hors dudit casier (81) ; et
un mécanisme de régulation de position (125) composé d'au moins une nervure (116) qui est formée dans un élément parmi ledit casier (81) et
ladite monture de chargement (82), et au moins une rainure d'engagement (92) qui est formée dans l'autre desdits éléments et
s'étend parallèlement audit canal d'insertion (91), ladite rainure d'engagement (92) étant configurée pour être engagée avec ladite nervure (116) afin de restreindre un mouvement vertical dudit casier (81) dans ledit canal d'insertion (91).

2. Paquet à pinces multiples (80) selon la revendication 1, dans lequel ladite nervure (116) se projette latéralement vers l'extérieur depuis un côté dudit casier (81), et ladite rainure d'engagement (92) est creusée latéralement depuis un côté dudit canal d'insertion (91).

3. Paquet à pinces multiples (80) selon la revendication 2, dans lequel ladite nervure (116) est intégrée dans une extrémité avant ou une extrémité arrière dudit casier (81).

4. Paquet à pinces multiples (80) selon l'une quelconque des revendications 1 à 3, dans lequel ledit casier (81) inclut un casier supérieur (112) et un casier inférieur (111), et dans lequel ledit casier supérieur (112) comprend une plaque-poussoir (113) intégrée à lui-même pour pousser ledit casier (81) contre ladite monture de chargement (82), et ledit casier inférieur (111) comporte lesdites nervures (116).

5. Paquet à pinces multiples (80) selon l'une quelconque des revendications 1 à 4, comprenant en outre :
une clavette (117) qui se projette depuis une face inférieure dudit casier (81) ; et
une gorge de clavette (93) dans ledit canal d'insertion (91), ladite gorge de clavette (93) recevant ladite clavette (117) pour empêcher une rotation axiale dudit casier (81).
